# EUROPÄISCHE PATENTSCHRIFT

(11) **EP 2 363 704 B1**
(45) Veröffentlichungstag und Bekanntmachung des Hinweises auf die Patenterteilung: **29.04.2015**
(21) Anmeldenummer: 11153973.0
(22) Anmeldetag: 10.02.2011
(51) Int. Cl.: G01N 27/28, G01N 27/416, C12M 1/36

(54) **Kalibrierbare Sensoreinheit für Reaktionsbehälter**
Calibratable sensor element for reaction container
Unité de détection pouvant être calibrée pour récipients de réaction

(30) Priorität: 10.02.2010 DE 102010001779
(43) Veröffentlichungstag der Anmeldung: 07.09.2011
(73) Patentinhaber: Hamilton Bonaduz AG, 7402 Bonaduz (CH)
(72) Erfinder: Löbbert, Andreas, 48249 Dülmen (DE); Schönfuss, Dirk, 7015 Tamins (CH)
(74) Vertreter: RLTG

(56) Entgegenhaltungen:
- EP-A2- 0 354 895
- EP-A2- 2 065 701
- WO-A1-2008/157181
- WO-A1-2009/059645
- US-A- 4 413 628
- US-A- 5 939 610
- US-A1- 2001 028 865
- US-A1- 2006 258 929

## Beschreibung

Die vorliegende Erfindung betrifft eine kalibrierbare Sensoreinheit für einen Reaktionsbehälter, wie etwa einen Fermenter, insbesondere Einwegfermenter, umfassend wenigstens eine zu kalibrierende Sensoreinrichtung und ein ein Kalibriermittel enthaltendes Kompartiment, wobei die wenigstens eine Sensoreinrichtung und das Kompartiment relativ zueinander beweglich in einem Gehäuse aufgenommen sind, das mit dem Reaktionsbehälter verbindbar ist, wobei die kalibrierbare Sensoreinheit dazu eingerichtet ist, die wenigstens eine Sensoreinrichtung durch Kontakt mit dem Kalibriermittel zu kalibrieren bevor mit der wenigstens einen Sensoreinrichtung chemische oder/und physikalische Parameter einer Messsubstanz gemessen werden, wobei die wenigstens eine Sensoreinrichtung relativ zu dem Kompartiment von einer Ausgangsposition, in welcher die Sensoreinrichtung bei bestimmungsgemäßem Gebrauch von der Messsubstanz nicht kontaktierbar ist, unter Änderung der Umgebung wenigstens eines Teils der Sensoreinrichtung, insbesondere eines sensitiven Elements, in eine von der Ausgangsposition verschiedene Messbereitschaftsposition beweglich ist, in welcher die Sensoreinrichtung wenigstens teilweise, insbesondere mit einem sensitiven Element, von der Messsubstanz kontaktierbar ist.

Nachfolgend ist mit einem Kompartiment wenigstens ein Kompartiment gemeint.

Eine ähnliche Sensoreinheit ist beispielsweise aus der EP 0 372 121 B1 bekannt. Die dort dargestellte Sensoreinrichtung kann in axialer Richtung einer sie aufnehmenden gehäuseartigen Hülse in einen Fermenter eingeführt und wieder herausgezogen werden. An ihrem in den Fermenter einzuführenden Ende weist die Sensoreinrichtung ein sensitives Element sowie eine Art Deckel auf, mittels welchem die Hülse beim Zurückziehen der Sensoreinrichtung gegenüber dem Fermenterinnenraum hermetisch abgedichtet werden kann. Wenn die Sensoreinrichtung vollständig in der Hülse eingefahren ist und somit gegenüber dem Fermenter abgedichtet ist, kann ein Reinigungsmittel oder ein Kalibriermittel durch an der Hülse angebrachte Zuführungen in die Hülse eingeleitet werden, so dass das sensitive Element der Sensoreinrichtung umspült werden kann, ohne dass Reinigungsmittel bzw. Kalibriermittel in den Fermenter austreten kann. Durch Anordnung von drei Dichtungsringen, die in axialer Richtung in vorbestimmten Abständen zueinander angeordnet sind, kann im Zusammenwirken mit verschiedenen Innenumfangswandungen unterschiedlicher Durchmesser eine Art Schleusenfunktion bereitgestellt werden. Diese Schleusenfunktion ermöglicht es, dass das Reinigungsmittel bzw. Kalibriermittel je nach Relativstellung der Sensoreinrichtung zum umgebenden Gehäuse unterschiedliche Bereiche im Inneren der Kalibriereinheit spülen kann. Der in der EP 0 372 121 B1 vorgeschlagene Sensor ist wiederverwendbar und kann mehrfach genutzt werden. Ferner ist er auch austauschbar in der Hülse bzw. dem Gehäuse der Kalibriereinheit aufgenommen.

Aus der US 5,939,610 ist eine gattungsgemäße Sensoreinheit mit einem aus einem hülsenartigen Gehäuse ausstoßbaren Sensor bekannt. Der Sensor ist in einem Kompartiment in Kalibrierflüssigkeit gelagert. Um den Sensor aus der umgebenden Hülse in Axialrichtung ausstoßen zu können, muss das Messgerät umgedreht werden, so dass das Ende, an welchem der Sensor austreten wird, im Wesentlichen vertikal nach oben gerichtet ist. Diese Ausrichtung des Messgeräts ist erforderlich, da ansonsten während des Ausstoßvorgangs des Sensors Kalibrierflüssigkeit aus dem Kompartiment herausfließen würde. Der Sensor kann mittels eines Feder-Rasten-Mechanismus mehrfach betätigt werden, ähnlich zu einer Betätigungseinrichtung bei einem Kugelschreiber. Wenn der Sensor bzw. sein sensitives Element so weit aus dem Gehäuse ausgestoßen worden ist, dass eine Messung vorgenommen werden kann, wird die Öffnung, durch welche der Sensor ausgetreten ist und welche durch einen schwenkbaren Deckel verschlossen und abgedichtet werden kann, durch das Zusammenwirken des Außenumfangs der Sensoreinrichtung und des Innenumfangs des Öffnungsrandes verschlossen, so dass kein Kalibriermittel während des Messvorgangs austreten kann. Aufgrund der möglichen mehrfachen Verwendung und Betätigung des Sensors besteht bei einem derartigen Messgerät die Gefahr, dass an dem sensitiven Element des Sensors anhaftende Verunreinigungen in die Kalibrierflüssigkeit gelangen können. Dies wird im übrigen noch dadurch begünstigt, dass auch beim Zurückziehen des Sensors in das Kompartiment mit Kalibrierflüssigkeit zurück das Messgerät in der oben beschriebenen vertikal nach oben gerichteten Stellung gehalten werden muss.

In der Biotechnologie und insbesondere auch in der Pharmaindustrie ist der Einsatz von so genannten Einwegfermentern gestiegen. Es werden immer mehr bekannte Stahlreaktoren durch Einwegfermenter aus Kunststoff ersetzt. Ein Beispiel für ein solches Einwegfermentersystem ist unter anderem der US 2005/0239199 A1 zu entnehmen. Der Einsatz von Einwegfermentern führt zu großen Zeit- und Kosteneinsparungen, da aufwändige Reinigungsschritte wie Clean In Process (CIP) und Sterilization In Process (SIP) entfallen, die bei Stahlreaktoren eine notwendige Maßnahme darstellen, um den Stahlfermenter für eine nächste Kultivierung zu entkeimen. Ein weiterer Vorteil von Einwegfermentern gegenüber konventionellen Stahlreaktoren ist der Ausschluss des Risikos von Kreuzkontaminationen, da Einwegfermenter vor ihrem Einsatz sterilisiert werden.

Um beim Einsatz von Einwegfermentern chemische und physikalische Parameter im Fermentationsgemisch, wie etwa pH-Wert, Leitfähigkeit, gelöster Sauerstoff, Zelldichte, optische Dichte, Druck, Kohlendioxidgehalt und andere Stoffkonzentrationen messen zu können, müssen auch an Einwegfermentern entsprechende, insbesondere physikalische, chemische, elektrochemische und/oder optische Sensoren vorgesehen werden. Voraussetzung für den Einsatz von Sensoren bei Einwegfermentern ist jedoch, dass diese in den Reaktionsraum (Innenraum) eines Einwegfermenters eingeführt werden müssen, ohne diesen beim Einführen zu kontaminieren. Dabei sind eine einfache Handhabung, niedrige Kosten und eine möglichst einfache, aber doch genaue Kalibrierbarkeit der Sensoren wünschenswert.

Zum Einführen von chemischen Sensoren in den Reaktionsraum von Einwegfermentern sind verschiedene Systeme vorgeschlagen worden. Beispielsweise werden optische Patches, etwa Polymermembranen, in denen optisch aktive Substanzen immobilisiert sind, in eine Wandung der Einwegfermenter integriert. Die in den Patches immobilisierten, optisch aktiven Substanzen ändern zum Beispiel in Abhängigkeit vom Partialdruck des gelösten Sauerstoffs oder des pH-Wertes des Fermentationsgemisches ihre Fluoreszenssignale, was mit entsprechenden von außen am Einwegfermenter angebrachten Anregungs-und Detektionsvorrichtungen gemessen und ausgewertet werden kann. Beispiel für derartige optische Systeme können der DE 101 37 530 A1 oder der US 2008/0032389 A1 entnommen werden. Problematisch bei der Verwendung derartiger Patches ist neben der eingeschränkten Auswahl an Sensoren die z.B. bei pH-sensitiven Patches geringe Stabilität der verwendeten optisch aktiven Substanzen gegenüber Gammastrahlen, die bei der Sterilisierung zum Einsatz kommen. Da weniger harte Bestrahlungen mit UVC- und Beta-Strahlen aufgrund ihrer geringen Eindringtiefen von nur wenigen Zehntel Millimetern häufig zu schwach sind, werden Einwegfermenter normalerweise mit Gammastrahlen bei ca. 50 kGrey zur Sterilisation bestrahlt. Ferner besteht bei derartigen Patches die Gefahr, dass deren optisch aktive Substanzen, die meist giftig und krebserregend sind, in den Fermenter freigesetzt werden und in der Folge dessen Inhalt kontaminiert wird. Weitere Probleme stellen sich durch die Ungewissheit über die Langzeitstabilität der Patches aufgrund von Alterung und die damit einhergehende fragliche Qualität der Vorkalibrierung dar, so dass in der Praxis in der Regel die externe Vermessung einer aus dem Einwegreaktor gezogenen Probe und ein Nachjustieren des Sensorsystems notwendig sind.

Aufgrund ihrer höheren Genauigkeit, sensorischen Vielfalt, ihres größeren Meßbereichs und ihrer größeren Robustheit ist es wünschenswert, dass auch traditionelle Sensoren, wie etwa die pH-Einstab-Messkette auch bei Einwegfermentern zum Einsatz kommen können. Eine Möglichkeit, traditionelle Sensoren bei Einwegfermentern einzusetzen, stellen Systeme dar, bei denen der Sensor in dem Reaktor integriert ist, so dass beide zusammen sterilisiert werden können. Hierzu wird beispielhaft auf die WO 2009/059645 A1 verwiesen. Die dort vorgeschlagene Vorrichtung ermöglicht zudem eine Einpunktkalibrierung, indem ein pH-Sensor in einem Behälter zusammen mit einer gammasterilisierbaren und pH-stabilen Aufbewahrungslösung integriert ist, die auch als Kalibrierlösung für eine Einpunktkalibrierung dient und mit dem Einwegfermenter vereint ist. Allerdings genügt für eine genaue pH-Messung mit kommerziellen pH-Sensoren eine Einpunktkalibrierung nicht aus. Hierdurch können Abweichungen von bis zu 0,25 pH-Einheiten auftreten. Da die Aufbewahrungslösung bzw. Kalibrierlösung beim vorgeschlagenen System beim Einbringen des Sensors in den Reaktionsraum (Innenraum des Fermenters) ebenfalls in diesen gelangt, kann dies zu einer Verunreinigung des Fermentationsgemisches führen, insbesondere bei kleinen Fermentervolumen.

Weitere Sensoreinheiten sind aus den Druckschriften EP 2065701 A2, US 4413628 A, US 2001/028865 A1, US 2006/258929 A1 bekannt.

Aufgabe der Erfindung ist es, eine kalibrierbare Sensoreinheit mit gegenüber dem Stand der Technik erhöhten Genauigheit und Zuverlässigkeit im Messergebnis bereitzustellen. Dabei ist das sensorische Prinzip des zu verwendenden Sensors unwesentlich.

Zur Lösung dieser Aufgabe wird vorgeschlagen, dass eine gattungsgemäße Sensoreinheit derart ausgebildet ist, dass die Relativbewegung zwischen der wenigstens einen Sensoreinrichtung und dem Kompartiment irreversibel durchführbar ist.

Da das Bewegen der Sensoreinrichtung, insbesondere ihres sensitiven Elements, von der Ausgangsposition in die Messbereitschaftsposition nicht rückgängig gemacht werden kann, kann das sensitive Element der Sensoreinrichtung mittels eines eindeutigen und festgelegten Ablaufs relativ zu dem Kompartiment bewegt werden. Hierdurch kann eine einfache und fehlerfreie Handhabung gewährleistet werden.

Unter einem Reaktionsbehälter wird jeder Behälter verstanden, in welchem eine Messsubstanz aufgrund chemischer oder/und biologischer Reaktionen oder/und aufgrund von Alterung Veränderungen unterworfen ist. Dabei können die Veränderungen durch Sensoren überwacht werden und gegebenenfalls Eingriffe in die im Reaktionsbehälter ablaufende Reaktion vorgenommen werden.

Durch ein bevorzugtes selbsttätiges Halten der Sensoreinrichtung in der Messbereitschaftsposition wird gewährleistet, dass das einmal in einen Reaktionsraum des Reaktionsbehälters (Fermenterinnenraum) eingebrachte sensitive Element dort verbleibt und nicht mehr in das Gehäuse der Kalibriereinheit zurückgeführt werden kann. Die Gefahr von Verunreinigungen bzw. von Kontamination ist dadurch minimal bzw. sogar verhindert. Bevorzugt kann die Sensoreinrichtung in der Messbereitschaftsposition insbesondere unüberwindbar verrastet sein.

Das Gehäuse der Sensoreinheit umgibt vorzugsweise die wenigstens eine Sensoreinrichtung in der Ausgangsposition und das Kompartiment. Hierdurch können die Sensoreinrichtung und das Kompartiment gegen Verunreinigung von außen geschützt werden.

Die Sensoreinrichtung, insbesondere ihr sensitives Element, kann in der Ausgangsposition in dem Kompartiment aufgenommen sein. Dies gewährleistet, dass das sensitive Element in einer chemisch günstigen Umgebung aufbewahrt werden kann vor seinem eigentlichen Einsatz im Fermenterinnenraum.

Um das Kompartiment abdichten zu können, kann es durch wenigstens ein Septum verschlossen sein. Dabei kann das Kompartiment mittels des Septums insbesondere vom durch das Gehäuse umschlossenen Innenraum der Sensoreinheit oder von einem benachbarten Kompartiment oder vom Fermenter getrennt werden. Vorzugsweise ist das wenigstens eine Septum für ein leichtes Durchdringen mittels der Sensoreinrichtung vorbereitet. Insbesondere kann das Septum Bereiche unterschiedlicher Materialdicke aufweisen oder/und es kann wenigstens teilweise perforiert sein. Eine Perforierung kann dabei das Septummaterial vollständig durchdringen oder aber als Teilperforation ausgeführt sein, also mit wenigstens einer Öffnung, die sich nicht über die gesamte Materialdicke des Septums erstreckt. Um das sensitive Element der wenigstens einen Sensoreinrichtung in das Kompartiment einführen zu können, wird in diesem Zusammenhang vorgeschlagen, dass die Sensoreinrichtung ein freies Ende aufweist, das derart ausgebildet ist, dass das wenigstens eine Septum des Kompartiments durchstoßbar ist, vorzugsweise in einer zur Ebene des Septums im Wesentlichen orthogonalen Richtung.

Das ein Kalibriermittel enthaltende Kompartiment kann als ein- und/oder beidseitig mit Septen verschlossene Einheit ausgebildet sein. Das wenigstens eine Septum kann durch bekannte Techniken, wie etwa Verkleben, Verschweißen, Verschrauben oder Klemmen, mit Wänden des Kompartiments verbunden werden.

Das wenigstens eine Septum ist vorzugsweise so ausgestaltet, dass das in dem Kompartiment enthaltene Kalibriermittel dauerhaft eingeschlossen wird, aber der Durchtritt des sensorischen Elements ermöglicht. Ferner ist es besonders vorteilhaft, wenn das wenigstens eine Septum derart ausgeführt ist, dass die Sensoreinrichtung bzw. das sensitive Element beim Durchdringen des wenigstens einen Septums gleichzeit vom Kalibriermittel gereinigt wird. Hierzu wird vorgeschlagen, dass das wenigstens eine Septum beispielsweise aus ein- oder mehrlagigen Folien oder Schichten aus elastischen Polymeren bestehen kann. Als Beispiele für mögliche Polymere werden PE, EVA und Polyamide genannt. Die verwendeten Polymere sollten alterungs- bzw. chemikalien- bzw. strahlungstabil und vorzugsweise auch FDA-konform sein. Alternativ zu den genannten Polymeren können auch Verbundwerkstoffe, Polymerblends oder Elastomere, wie z. B. eine (FDA-geprüfte) Moosgummifolie, Anwendung finden, die vorher zur leichteren Durchdringung präpariert werden können, beispielsweise durch Bereiche unterschiedlicher Materialdicke, eine (Teil-)Perforation oder dergleichen. Hierdurch kann eine Art Sollbruchstelle in einem betreffenden Septum bereitgestellt werden.

Die Sensoreinheit kann mehrere Kompartimente umfassen, die bezogen auf die Durchstoßrichtung der Sensoreinrichtung hintereinander angeordnet und durch jeweilige dazwischen angeordnete Septen voneinander getrennt sind. Dies ermöglicht beispielsweise das Anordnen von mehreren Kompartimenten hintereinander in der Richtung, in welcher die Sensoreinrichtung von der Ausgangsposition in die im Fermenter eingeführte Endposition bewegt wird. Da die einzelnen Kompartimente durch Septen voneinander getrennt sind, wird gewährleistet, dass sich die in den Kompartimenten befindlichen Kalibriermittel nicht mischen können. Ferner kann durch die Septen das Abstreifen von Kalibriermitteln des einen Kompartiments gewährleistet werden, bevor das sensitive Element in das benachbarte Kompartiment mit einem anderen Kalibriermittel eingeführt wird.

Alternativ oder ergänzend kann die Sensoreinheit mehrere Kompartimente umfassen, die im Wesentlichen orthogonal zur Durchstoßrichtung nebeneinander angeordnet sind. Bei einer derartigen Anordnung von Kompartimenten kann das sensitive Element der Sensoreinrichtung aus einem ersten Kompartiment herausgeführt werden und dann nach einer Zustellbewegung zu einem nächsten Kompartiment in dieses eingeführt werden.

Sofern mehrere Kompartimente bei einer Sensoreinheit vorgesehen sind, kann in wenigstens einem dieser Kompartimente ein Reinigungsmittel enthalten sein, mittels welchem das sensitive Element der Sensoreinrichtung von anhaftenden Verunreinigungen gereinigt werden kann, um so zu gewährleisten, dass keine durch Verunreinigungen verursachte Messwertfehler oder gegebenenfalls Kontaminationen im Reaktionsbehälter, etwa Fermenter, auftreten.

Um das Anhaften von Verunreinigungen noch weiter zu unterdrücken, wird vorgeschlagen, dass dem Kompartiment eine Abstreifeinrichtung zugeordnet ist, die von der Sensoreinrichtung durchstoßbar ist und derart ausgebildet ist, dass an der Sensoreinrichtung anhaftende Verunreinigungen, insbesondere anhaftendes Kalibriermittel oder gegebenenfalls Reinigungsmittel, im betreffenden Kompartiment mit Kalibriermittel oder gegebenenfalls Reinigungsmittel zurückbehalten wird. Wie bereits oben angedeutet, kann die Abstreifeinrichtung durch wenigstens ein Septum bzw. durch das wenigstens eine Septum oder durch wenigstens ein zusätzliches in Durchstoßrichtung dem Septum benachbartes Abstreifmittel, insbesondere einen Dichtring, gebildet sein.

Vorzugsweise umfasst die kalibrierbare Sensoreinheit auf die wenigstens eine Sensoreinrichtung wirkende Betätigungsmittel, die derart ausgeführt sind, dass mittels der Betätigungsmittel die irreversible Relativbewegung zwischen der wenigstens einen Sensoreinrichtung und dem Kompartiment durchführbar ist. Als Betätigungsmittel können von außerhalb des Gehäuses manipulierbare Bauteile zum Einsatz kommen, die über mechanische Verbindungen unterschiedlichster Art mit der Sensoreinrichtung bzw. dem Gehäuse verbunden sein können, so dass durch das Zusammenwirken der mechanischen Verbindungen eine irreversible Führung der Sensoreinrichtung mittels des Betätigungsmittels erfolgt. Derartige mechanische Verbindungen können beispielsweise Gewinde, Feder-Nut-Eingriffe, Verrastungen oder dergleichen sein.

Das Kalibriermittel oder/und das Reinigungsmittel ist vorzugsweise als sterilisierbares viskoses Medium ausgeführt, insbesondere als Gel oder Paste. Derartige insbesondere hochviskose Kalibriermittel sind formstabil, und im Gegensatz zu Flüssigkeiten verlaufen sie nicht. Dies kann von besonderem Vorteil sein, wenn in einem Kompartiment noch eine Gasphase enthalten ist. Eine an dem sensitiven Element befindliche Gasphase kann die Kalibrierung stören oder gar unmöglich machen. Eine komprimierbare Gasphase in einem Kompartiment ist jedoch erforderlich, um die Verdrängung von Kalibriermittel durch den in das Kompartiment eintauchenden Sensor zu kompensieren, sofern das betreffende Kompartiment mit starren Wänden vorgesehen ist. In einem solchen Fall ist es bevorzugt, dass das Verhältnis von Gelphase zu Gasphase so gewählt ist, dass der Sensor in jeder Lage ausreichend mit Kalibriermittel benetzt bleibt. Hierdurch ist es möglich, die kalibrierbare Sensoreinheit in jeder beliebigen Ausrichtung der Sensoreinrichtung zu verwenden.

Falls weniger viskose, noch fließfähige Kalibriermittel bzw. Reinigungsmittel verwendet werden sollen, ist es denkbar, eine Gasphase in dem Kompartiment zu vermeiden. Um den Volumenzuwachs, der durch das Einbringen der Sensoreinrichtung in das Kompartiment entsteht, kompensieren zu können, kann das Kompartiment flexible Wände aufweisen, beispielsweise in Form einer Art Faltenbalg.

Das wenigstens eine Kalibriermittel kann ein synthetisches Polymer, insbesondere Polyvinylalkohol oder Hydroxyethylcellulose, oder natürliche Polymere, insbesondere Polysaccharide als Verdickungsmittel enthalten.

Die Erfindung betrifft nach einem weiteren Aspekt auch einen Reaktionsbehälter, wie etwa Fermenter, insbesondere Einwegfermenter, mit einem Reaktionsraum und einer damit fest verbundenen, insbesondere verschraubten, verklebten oder verschweißten Kalibriereinheit mit wenigstens einem der oben genannten Merkmale, wobei der Reaktionsbehälter und die kalibrierbare Sensoreinheit gemeinsam sterilisierbar oder sterilisiert sind.

Weiterbildend wird vorgeschlagen, dass bei einem Reaktionsbehälter die Sensoreinrichtung in der bereits angesprochenen Messbereitschaftsposition wenigstens teilweise, insbesondere mit ihrem sensitiven Element im Reaktionsraum aufgenommen ist.

Der Reationsraum kann durch wenigstens ein dem Reaktionsbehälter oder/und der kalibrierbaren Sensoreinheit zugeordnetes Septum von der kalibrierbaren Sensoreinheit getrennt sein. Dies ermöglicht einerseits eine Abdichtung zwischen Reaktionsraum und der kalibrierbaren Sensoreinheit und andererseits einen Zugang für die Sensoreinrichtung in den Reaktionsraum beim Durchdringen des Septums.

Ferner wird auch ein Verfahren zum Kalibrieren wenigstens einer Sensoreinrichtung einer mit einem Reaktionsbehälter, wie etwa Fermenter, insbesondere Einwegfermenter, verbundenen kalibrierbaren Sensoreinheit mit wenigstens einem der oben genannten Merkmale vorgeschlagen, wobei dieses Verfahren folgende Schritte umfasst:
a) Einführen eines sensitiven Elements der wenigstens einen Sensoreinrichtung in ein Kalibriermittel enthaltendes Kompartiment der kalibrierbaren Sensoreinheit,
b) Herausführen des sensitiven Elements aus dem Kompartiment,
c) Einführen des sensitiven Elements in den Reaktionsbehälter und Arretieren der Sensoreinrichtung,
wobei die Schritte a) bis c) irreversibel durchgeführt werden.

Weiterbildend wird zum Verfahren vorgeschlagen, dass die Schritte a) und b) vor Ausführung des Schritts c) an wenigstens einem weiteren Kompartiment durchgeführt werden, in dem ein weiteres Kalibriermittel oder ein Reinigungsmittel enthalten ist.

Beim Wiederholen der Schritte a) und b) an jedem weiteren Kompartiment wird die wenigstens eine Sensoreinrichtung so geführt, gegebenenfalls temporär arretiert, dass das sensitive Element nicht mehr in Kontakt mit einem vorhergehenden Kompartiment kommt.

Die Erfindung wird nachfolgend beispielhaft und nicht einschränkend unter Bezugnahme auf die anliegenden Figuren anhand zweier Ausführungsformen beschrieben.
- Figur 1: zeigt in schematischer und perspektivischer Explosionsdarstellung eine kalibrierbare Sensoreinheit gemäß einer ersten Ausführungsform der Erfindung.
- Figur 2: zeigt in den Teilfiguren a) bis c) die Sensoreinheit der Figur 1 in einer Längsschnittdarstellung in verschiedenen Positionen bei deren Verwendung.
- Figur 3: zeigt eine schematische und perspektivische Explosionsdarstellung einer zweiten Ausführungsform der erfindungsgemäßen Sensoreinheit.
- Figur 4 bis 10: zeigen Schnittdarstellungen der Sensoreinheit der Figur 3 in unterschiedlichen Positionen während deren Verwendung.
- Figur 11: zeigt eine schematische Draufsicht auf die Sensoreinheit der Figur 3.

Die Erfindung wird nachfolgend anhand von zwei Ausführungsformen näher erläutert, wobei rein beispielhaft als Sensoren so genannte pH-Einstab-Messketten beschrieben werden. Dies stellt in keiner Weise eine Einschränkung der Sensoreinheit auf derartige Sensoren dar, sondern es können ebenso gut andere Sensoren zur Messung weiterer chemischer bzw. physikalischer Eigenschaften vorgesehen werden. Ferner wird auch darauf hingewiesen, dass die in den Ausführungsbeispielen dargestellte Anzahl von Sensoren, die bei der jeweiligen Sensoreinheit beschrieben werden, ebenfalls nicht einschränkend anzusehen ist, sondern dass neben den dargestellten Sensoreinheiten mit einem bzw. zwei Sensoren auch mehr Sensoren in einer Sensoreinheit vorgesehen sein können. Ferner wird darauf hingewiesen, dass an einem Reaktionsbehälter, wie etwa Fermenter, insbesondere Einwegfermenter nicht nur eine der nachfolgenden Ausführungsformen der Sensoreinheit angeordnet werden kann, sondern dass auch mehrere solcher Sensoreinheiten, gegebenenfalls auch unterschiedliche Typen von Sensoreinheiten an gewünschten Stellen eines Reaktionsbehälters angebracht sein können. Nachfolgend wird als Reaktionsbehälter ein Fermenter beschrieben, was aber keinesfalls einschränkend zu verstehen ist.

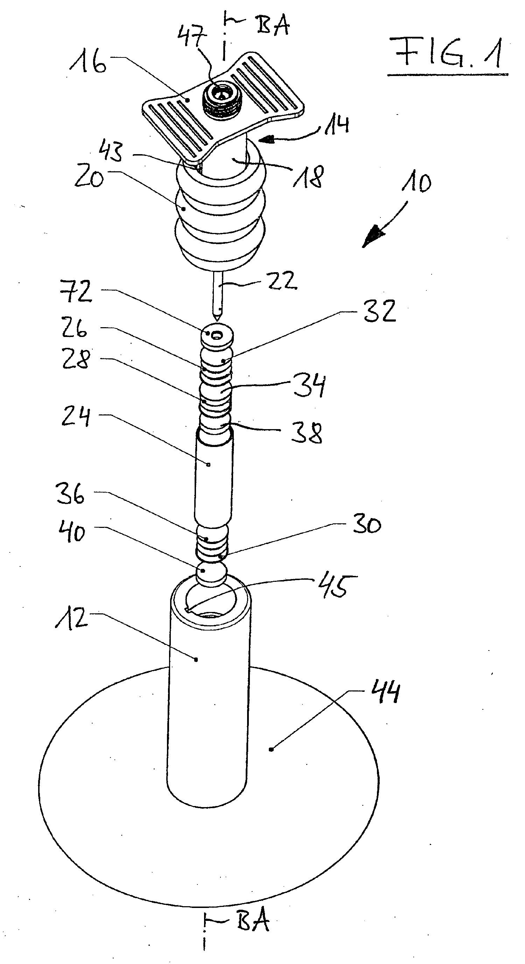

Eine in Figur 1 schematisch dargestellte erste Ausführungsform einer Sensoreinheit 10 umfasst ein hülsenartiges bzw. stutzenartiges Gehäuse 12, das in seiner Längsrichtung eine Bewegungsachse BA festlegt, entlang welcher eine Sensoreinrichtung 14 bewegt werden kann. Die Sensoreinrichtung 14 umfasst an ihrem oberen Ende ein plattenartiges oder flügelartiges Betätigungselement 16, das außerhalb des Gehäuses 12 angeordnet ist und von einem Anwender ergriffen werden kann. Das Betätigungselement 16 ist mit einer Führungshülse 18 verbunden, die sich im Wesentlichen orthogonal zum Betätigungselement 16 in Richtung BA erstrecken kann. Um die Führungshülse herum kann zur Abdichtung des Gehäuses 12 ein Dichtungselement 20, hier beispielhaft ein Faltenbalg 20, angeordnet sein, um das Innere des Gehäuses 12 gegenüber der Umgebung abzudichten. Mit der Führungshülse 18 bzw. dem Betätigungselement 16 ist ein Sensor 22 verbunden. Dieser Sensor 22 ist, wie bereits einleitend zur Figurenbeschreibung erwähnt, als ph-Einstab-Messkette ausgeführt. Im Gehäuse 12 ist eine Aufnahmehülse 24 angeordnet, welche dafür vorgesehen ist, mehrere, im vorliegenden Beispiel drei Kompartimente 26, 28 und 30 aufzunehmen. Die Kompartimente 26, 28 und 30 sind mittels Septen 32, 34 und 36 voreinander getrennt bzw. abgedichtet. Zwischen den Kompartimenten 28 und 30 sowie in Richtung BA unten anschließend an das Kompartiment 30 sind so genannte Reinigungssepten 38 bzw. 40 angeordnet, welche dazu dienen, am Sensor 22 anhaftende Verunreinigung beim Durchstoßen durch das betreffende Septum 38, 40 zurückzuhalten, so dass keine Verunreinigungen in das nächste Kompartiment (hier Kompartiment 30) bzw. dann in den nicht dargestellten Fermenter gelangt, der sich in Richtung BA unten an die Kalibriereinheit 10 anschließt.

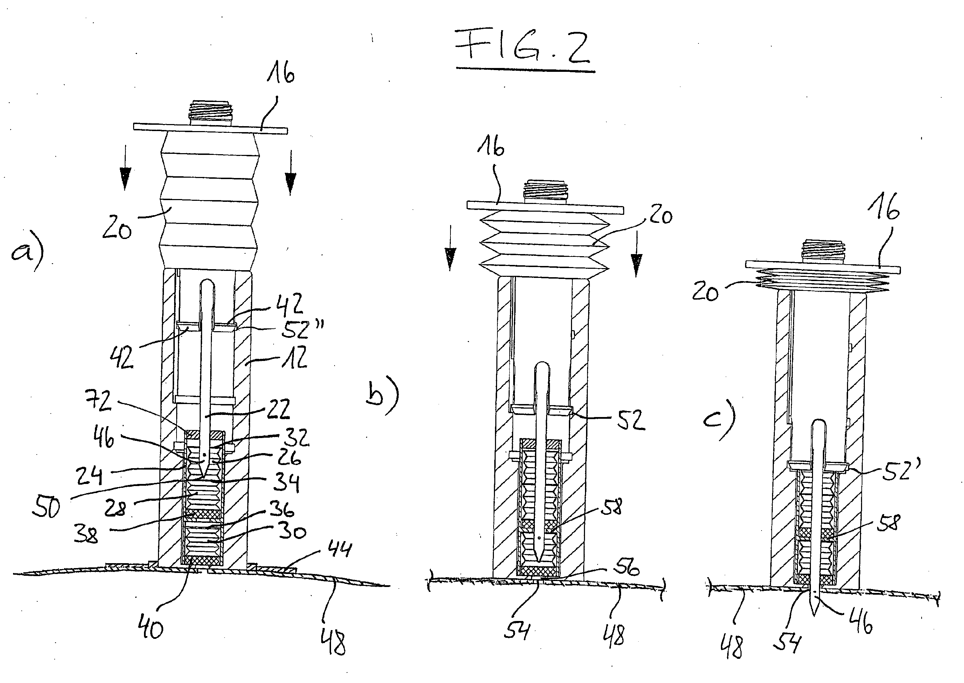

Die Aufnahmehülse 24, das Gehäuse 12 und die Führungshülse 18 sind so dimensioniert, dass die Sensoreinrichtung 14 relativ zur Aufnahmehülse 24 bzw. den darin aufgenommenen Kompartimenten 26, 28, 30 in Richtung BA von oben nach unten bezogen auf die gewählte Darstellung bewegt werden kann. Beim Bewegen der Sensoreinrichtung 14 relativ zur Aufnahmehülse 24 und zum Gehäuse 12 wird die Führungshülse 18 in radialer Richtung zwischen der Aufnahmehülse 24 und dem Gehäuse 12 aufgenommen. An der Führungshülse 18 sind in unteren Endbereichen insbesondere federnde Rastvorsprünge 42 (Figur 2a) vorgesehen, welche in entsprechende Rastausnehmungen eingreifen können, die an einer Innenumfangswand des Gehäuses 12 vorgesehen sein können. Um ein Verdrehen der Führungshülse 18 um die Achse BA zu verhindern steht sie mit einem bei 43 angedeuteten Vorsprung in Eingriff mit einer im Gehäuse 12 ausgebildeten Führungsnut 45. Ferner wird noch darauf hingewiesen, dass die Sensoreinrichtung 14 an ihrem oberen Ende (vom Fermenter abgewandt) ein Anschlussstück 47 aufweist, an welchem der Sensor mit einer Messvorrichtung oder dergleichen verbunden werden kann.

An ihrem einem nicht dargestellten Fermenter zugeordneten Ende (in der Figur 1, unteres Ende, bezogen auf die Richtung BA) weist die Sensoreinheit 10 ein tellerartiges Befestigungselement 44 auf, mittels welchem die Sensoreinheit 10 mit einem Fermenter verbunden werden kann. Bevorzugt ist das Befestigungselement 44 als eine Art Anschweißlippe ausgebildet, die mit dem Fermenter verklebt bzw. verschweißt werden kann. Dabei ist es besonders bevorzugt, wenn sowohl die Anschweißlippe 44 als auch der Fermenter aus einem Kunststoff hergestellt sind, was insbesondere bei so genannten Einwegfermentern der Fall sein kann. Denkbar ist auch eine Verschraubung oder andersartige lösbare Verbindung zwischen Sensoreneinheit und Fermenter.

Figur 2 zeigt in den Teilfiguren a) bis c) das Bewegen des Sensors 22 von einer Ausgangsposition (Figur 2a) über eine Zwischenposition (Figur 2b) in eine Messbereitschaftsposition (Figur 2c), in welcher der Sensor 22 mit seinem unteren bzw. vorderen sensitiven Bereich bzw. sensitiven Element 46 in einem Innenraum (Reaktionsraum) eines hier nur angedeuteten Fermenters 48 (Reaktionsbehälter) angeordnet ist.

In der Ausgangsposition gemäß Figur 2a) befindet sich der Sensor 22 mit seinem sensitiven Element 46 im Kompartiment 26, das mit einem Kalibriermittel gefüllt ist, beispielsweise einem pH-Puffergel pH4. In dieser Ausgangsposition kann eine Kalibrierung des Sensors 22 auf Grundlage des im Kompartiment 26 aufgenommenen Puffergels durchgeführt werden. Nach Durchführen dieses ersten Kalibrierungsschrittes wird der Sensor 22 mittels des Betätigungselements 16 in Richtung des Fermenters 48 bewegt. Dabei durchstößt der Sensor 22 mit seinem dem Fermenter 48 zugewandten Ende 50, das als Spitze ausgeführt sein kann, das Septum 34, welches das Kompartiment 26 vom Kompartiment 28 trennt bzw. abdichtet. Im Kompartiment 28 kann sich ein Reinigungsmittel befinden, so dass der Sensor 22, insbesondere dessen sensitives Ende 46, von Verunreinigungen mit Kalibriermittel aus dem Kompartiment 26 gereinigt werden kann. Beim weiteren Bewegen des Betätigungselements 16 und des Sensors 22 zusammen mit der Führungshülse 18 zum Fermenter 48 hin durchstößt die Spitze 50 das Reinigungsseptum 38 sowie das das Kompartiment 30 verschließende Septum 36, so dass das sensitive Element 46 sich gemäß Figur 2b) im Kompartiment 30 befindet. In dieser Stellung erfolgt ein Verrasten der Rastvorsprünge 42 der Führungshülse 18 mit im Gehäuse 12 vorgesehenen Rastausnehmungen 52. Die Rastausnehmungen können auch in Form einer Art Ringnut als eine einzige Rastausnehmung 52 ausgebildet sein. Die Rastvorsprünge 42 sind vorzugsweise derart ausgebildet, dass sie mit den Rastausnehmungen 52 so in Eingriff stehen, dass eine Bewegung des Betätigungselements 16 und somit des Sensors 22 vom Fermenter 48 weg verunmöglicht bzw. nur mit hohem Kraftaufwand möglich ist. Insoweit stellt das Zusammenwirken von Rastvorsprüngen 42 und Rastausnehmungen 52 eine Art Arretierung der Sensoreinrichtung 14 relativ zu den Kompartimenten 26, 28, 30 dar. Da die Rastvorsprünge 42 zum Fermenter hin vorzugsweise eine konisch ausgebildete Umfangsfläche aufweisen, können sie in Richtung des Fermenters 48 unter elastischer radialer Auslenkung nach innen aus der Rastausnehmung 52 geführt werden.

Im Kompartiment 30 kann sich ein weiteres Kalibriermittel, etwa ein pH7-Puffergel befinden, so dass in der in Figur 2b) dargestellten Zwischenstellung eine weitere Kalibrierung des Sensors 22 vorgenommen werden kann.

Nachdem diese zweite Kalibrierung vorgenommen worden ist, kann das Betätigungselement 16 weiter in Richtung des Fermenters 48 bewegt werden, so dass der Sensor 22, insbesondere sein sensitives Element 46, durch das Reinigungsseptum 40 hindurch und durch eine im Fermenter 48 vorgesehene Öffnung 54 hindurch in den Innenraum des Fermenters 48 bewegt werden kann. In diesem Zusammenhang wird darauf hingewiesen, dass auch das Gehäuse 12 eine für den Durchtritt des Sensors 22 vorgesehene, zur Öffnung 54 des Fermenters 48 benachbarte Öffnung 56 aufweisen kann. Diese kann ebenfalls durch ein Septum verschlossen sein.

Wie in Figur 2c) dargestellt, greifen die Rastvorsprünge 42 der Führungshülse 18 in der dargestellten Endposition des Sensors 22 ebenfalls in eine bzw. mehrere Rastausnehmungen 52' ein, so dass das Betätigungselement 16 und damit der Sensor 22 nicht in Richtung weg vom Fermenter 48 bewegt werden kann.

Es wird darauf hingewiesen, dass in der dargestellten Ausführungsform die Kompartimente 26, 28 und 30 als Faltenbalg ausgeführt sind und vollständig mit dem jeweiligen Kalibriermittel bzw. Reinigungsmittel gefüllt sind, ohne dass sich im betreffenden Kompartiment eine Gasphase befindet. Beim Eindringen des Sensors 22 in die Kompartimente dehnen sich diese aufgrund der Verdrängung des Kalibrier- bzw. Reinigungsmittels in Längsrichtung innerhalb der Aufnahmehülse 24 aus. Es wird ferner darauf hingewiesen, dass die Aufnahmehülse 24 einen radial nach innen weisenden Vorsprung 58 aufweisen kann, auf welchem das Reinigungsseptum 38 aufliegt. Das Kompartiment 30 ist somit zwischen diesem Vorsprung 58 und dem unteren, die Öffnung 56 bildenden Rand des Gehäuses 12 aufgenommen.

Die Bewegung des Sensors 22 bzw. seines sensitiven Elements 46 von der Ausgangsposition gemäß Figur 2a) in die Endposition gemäß Figur 2c) erfolgt unumkehrbar, so dass das sensitive Element 46 wohl definiert vom ersten Kompartiment 26 in das zweite Kompartiment 28, das dritte Kompartiment 30 und schließlich in den Innenraum des Fermenters 48 bewegt werden kann. Ein Bewegen des Sensors 22 vom Fermenter 48 weg ist verhindert durch den Eingriff zwischen den Rastvorsprüngen 42 der Führungshülse 18 und den Rastausnehmungen 52 bzw. 52' im Gehäuse 12. Es wird in diesem Zusammenhang noch darauf hingewiesen, dass die Rastvorsprünge 42 auch in der Ausgangsstellung gemäß Figur 2a) in einer Rastausnehmung 52" aufgenommen sein können, so dass der Sensor 22 bzw. das sensitive Element 46 sicher in dieser Ausgangsstellung gehalten wird und das sensitive Element 46 in der Pufferlösung des Kompartiments 26 aufgenommen ist.

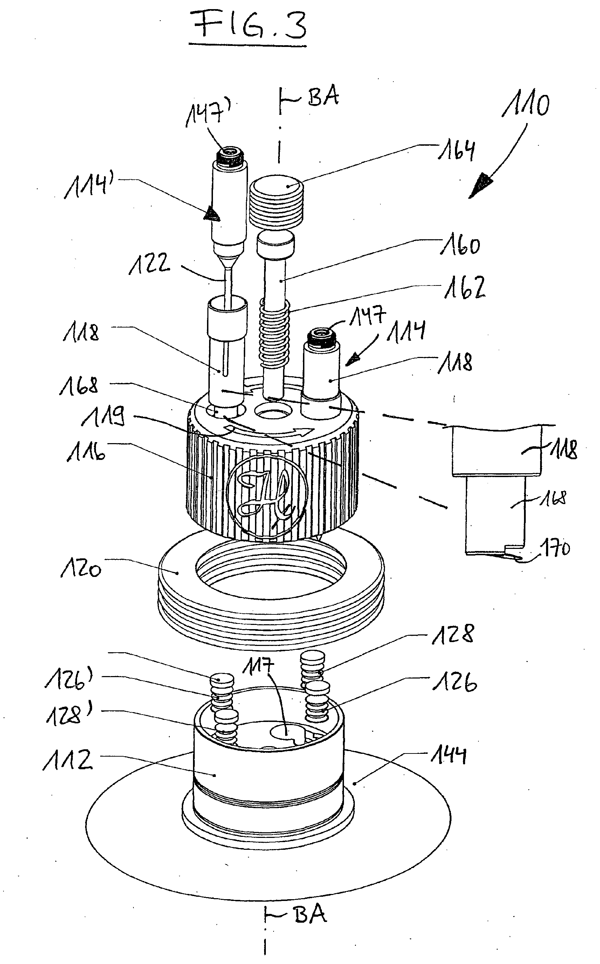

Figur 3 zeigt eine zweite Ausführungsform einer Sensoreinheit, wobei zur ersten Ausführungsform ähnliche bzw. gleiche Bauteile mit den gleichen um 100 erhöhten Bezugszeichen bezeichnet sind.

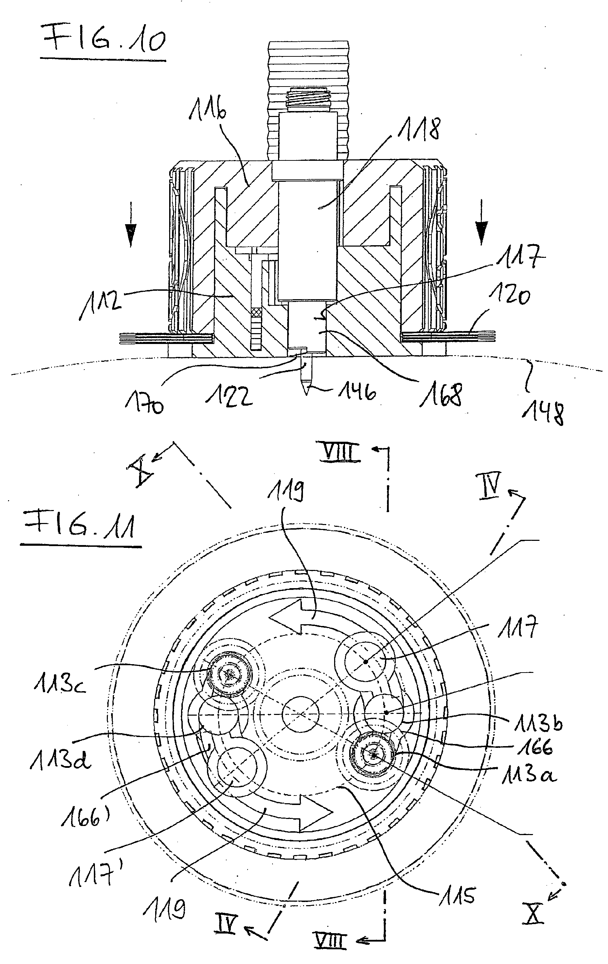

Eine Sensoreinheit 110 gemäß einer zweiten Ausführungsform umfasst ein Gehäuse 112, das vorzugsweise stutzen- bzw. rohrartig ausgeführt ist, wobei im Inneren des Gehäuses Aufnahmen vorgesehen sind, in denen Kompartimente 126, 126', 128 und 128' aufgenommen werden können. Die Aufnahmen sind beispielhaft aus der Figur 11 ersichtlich und mit den Bezugszeichen 113a bis 113d versehen. Wie sich aus der Zusammenschau von Figur 3 und Figur 11 ergibt, sind die Kompartimente 126, 126', 128 und 128' entlang einer bogenförmigen, vorzugsweise kreisförmigen, Linie 115 nebeneinander angeordnet. Dabei bilden die Kompartimente 126, 128 und 126', 128' jeweilige Gruppen von Kompartimenten für eine jeweilige Sensoreinrichtung 114, 114' (Figur 3). Im Gehäuse 112 sind ferner benachbart zu den Aufnahmen 113b bzw. 113d Durchgänge 117 bzw. 117' vorgesehen, durch welche hindurch die jeweilige Sensoreinrichtung 114, 114' in einen zugeordneten Fermenter bewegt werden kann.

Die Sensoreinrichtungen 114, 114' sind in einem Betätigungselement 116 aufgenommen und vorzugsweise fest mit diesem verbunden. Das Betätigungselement 116 ist vorzugsweise als eine Art Kappe ausgebildet, die relativ zum Gehäuse 112 gedreht werden kann in Richtung der Pfeile 119 (hier beispielhaft im Gegenuhrzeigersinn). Die Drehung erfolgt um eine mit dem Gehäuse 112 verbundene Achse bzw. Welle 160. Entlang der Axialrichtung BA der Welle 160 kann das Betätigungselement 116 nach oben bzw. unten, also vom Fermenter weg bzw. zum Fermenter hin, bewegt werden. Bevorzugt wird das Betätigungselement 116 mittels einer Feder 162 in Richtung des Fermenters vorgespannt.

Eine Abdichtung zwischen dem Betätigungselement 116 und dem Gehäuse 112 erfolgt durch einen Faltenbalg 120, und eine weitere Abdichtung zwischen der feststehenden Welle 160 und dem Betätigungselement 116 erfolgt mittels eines weiteren Faltenbalgs 164. Auch in diesem Ausführungsbeispiel kommen als Sensoren beispielhaft pH-Einstab-Messketten 122 zum Einsatz. Wie aus der Figur 3 ersichtlich, sind in dem Betätigungselement 116 zwei Sensoreinrichtungen 114, 114' vorgesehen, die mittels des Betätigungselements 116 gemeinsam in Bewegung versetzt werden können. Es wird darauf hingewiesen, dass es sich bei den Sensoreinrichtungen 114, 114' auch um unterschiedliche Sensoren handeln kann, beispielsweise einen pH-Sensor und einen Sensor zur Messung des Sauerstoffgehalts oder dergleichen.

Das Verdrehen des Betätigungselements 116 in Pfeilrichtung 119 (hier Gegenuhrzeigersinn) ist vorzugsweise nur ermöglicht, wenn das Betätigungselement 116 entgegen der durch die Feder 162 wirkenden Vorspannung nach oben bewegt wird. Ferner sind die Ausnehmungen 113a bis 113d, die Durchgänge 117, 117' durch Nuten bzw. Führungen 166 bzw. 166' so miteinander verbunden, dass im Zusammenwirken mit den die Sensoren 122 umgebenden jeweiligen Hülsen 118 (bzw. Hülse 118' nicht sichtbar) ein Verdrehen des Betätigungselements 116 in der Gegenrichtung (hier Uhrzeigersinn) verhindert ist. Durch die Drehbewegung und das Anheben bzw. Absenken des Betätigungselements 116 können die Sensoreinrichtungen 114, 114' den jeweils zugeordneten Kompartimenten 126, 128 bzw. 126', 128' zugeführt werden. Ferner kann in einem letzten Schritt die Zuführung der Sensoreinrichtung 114, 114' zum Fermenter hin erfolgen durch die Durchgänge 117, 117' hindurch.

Es wird darauf hingewiesen, dass am Gehäuse 112 eine Anschweißlippe 144 vorgesehen ist, mittels welcher die Sensoreinheit 110 mit einem zugehörigen Fermenter, vorzugsweise Einwegfermenter, verbunden werden kann. Ferner weisen die Sensoreinrichtungen 114, 114' jeweilige Anschlussstücke 147, 147' auf für die Verbindung mit einem Messgerät oder dergleichen.

Die den Sensor 122 aufnehmende Führungshülse 118 weist in einem unteren Abschnitt eine Art Gleitzapfen 168 auf, der durch die Ränder bzw. Nuten 166, 166' geführt wird. Der Gleitzapfen 168 weist ein nach unten bzw. zum Fermenter hin vorgespanntes elastisches Arretierelement 170 auf, das durch Zusammenwirken mit im Gehäuse 112 ausgebildeten Führungen bzw. Nuten 166, 166' bzw. Umfangsrändern der Aufnahmen 113a bis 113d ein Verdrehen des Betätigungselements 116 entgegen der Pfeilrichtung 119 verhindern kann.

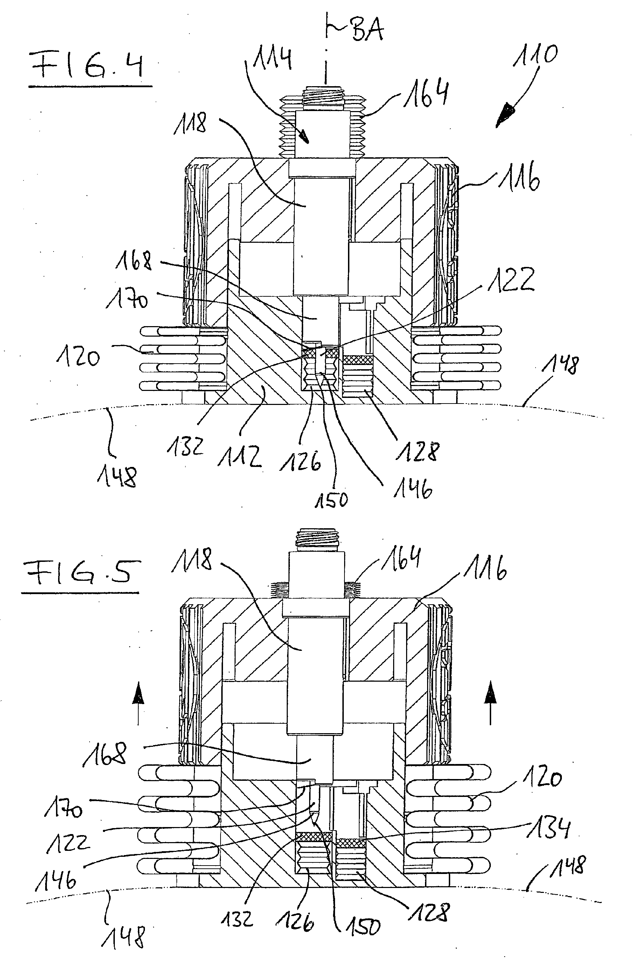

Nachfolgend werden unter Bezugnahme auf die Figuren 4 bis 10 am Beispiel der Sensoreinrichtung 114 der Bewegungsablauf und der Kalibriervorgang beschrieben werden. Dabei stellen die Figuren 4 bis 10 tangential zum Bogen 115 verlaufende Schnitte dar, wie dies durch die Schnittlinien IV-IV, VIII-VIII und X-X exemplarisch in der Figur 11 dargestellt ist. Die in den Figuren 5 bis 7 und 9 dargestellten Schritte sind ebenfalls Tangentialschnitte an Zwischenpositionen, und es wurde aus Gründen der Übersichtlichkeit auf entsprechende weitere Schnittlinien in der Figur 11 verzichtet.

Figur 4 zeigt die zusammengesetzte Sensoreinheit 110 in einer so genannten Ausgangsposition. In dieser Ausgangsposition ist der Sensor 122, insbesondere sein sensitives Element 146 im Kompartiment 126 aufgenommen. In diesem Kompartiment 126 kann sich ein Kalibriermittel befinden, wie etwa eine pH-Pufferlösung. Das Kompartiment 126 ist zur Innenseite des Gehäuses 112 bzw. der Sensoreinheit 110 mitttels eines durch den Sensor 122 durchstoßbaren Septums 132 verschlossen. Das Septum 132 kann auch eine reinigende Wirkung haben, so dass beim Herausziehen des Sensors 122 aus dem Kompartiment 126 am Sensor anhaftende Verunreinigungen im Kompartiment 126 weitestgehend zurückbehalten werden können. Wie aus der Figur weiter ersichtlich ist, kann das Betätigungselement in dieser hier dargestellten Ausgangsposition nicht im Uhrzeigersinn um die Achse BA verdreht werden, da das an der Hülse 118 bzw. am Gleitzapfen 168 vorgesehene Arretierelement 170 an einem diese Drehung verhindernden Rand des Gehäuses 112 anliegt.

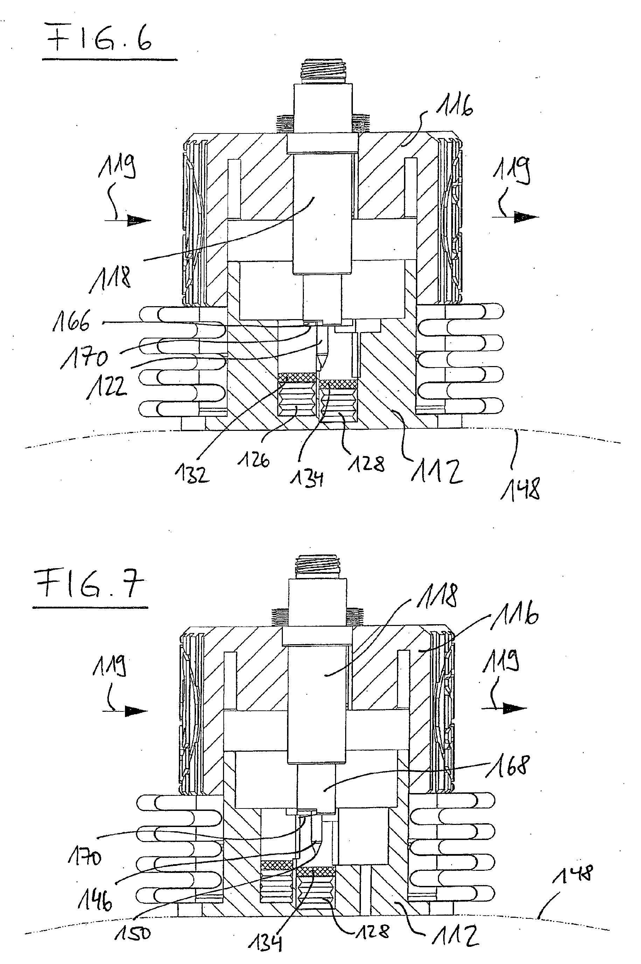

Um den Sensor 122 für Messungen im Fermenter 148 in Betrieb zu nehmen, kann nach dem Durchführen einer ersten Kalibrierung im Kompartiment 126 das Betätigungselement 116 entlang der Achse bzw. Welle 160 (Figur 3) nach oben, also vom Fermenter 148 weg, bewegt werden, so dass mit ihm die Führungshülse 118 und der Sensor 122 nach oben bewegt werden. Nachdem das Betätigungselement 116 um einen bestimmten Betrag nach oben bewegt worden ist, kann, wie dies in der Figur 6 dargestellt ist, eine Drehbewegung um die Achse bzw. Welle 160 (Figur 3) in Richtung der Pfeile 119 (Gegenuhrzeigersinn) erfolgen, wobei der Sensor 122 von der Aufnahme 113a hin zur Aufnahme 113b (Figur 11) entlang der Bewegungslinie 115 bewegt werden kann. Bei dieser Drehbewegung ist, wie dies aus der Figur 6 ersichtlich ist, das Arretierelement 170 in etwa horizontaler Anlage auf einem Führungsrand bzw. einer Nut 166, die zwischen den beiden Aufnahmen 113a, 113b ausgebildet ist. Sobald der Sensor 122 in etwa mit der Aufnahme 113b fluchtet (Figur 7) wird das Arretierelement 170 aufgrund seiner elastischen Vorspannung in die Aufnahme 113b bewegt und hintergreift deren Innenrand, so dass eine Drehbewegung entgegen der in den Figuren 6 und 7 gezeigten Pfeilrichtung 119 nicht mehr möglich ist. Nach Beenden der Drehbewegung von der ersten Aufnahme 113a zur zweiten Aufnahme 113b hin erfolgt ein Absenken des Betätigungselements 116 in Richtung des Fermenters 148, so dass der Sensor 122, insbesondere sein sensitives Element 146 in das Kompartiment 128 bewegt werden kann. Bei dieser Zustellbewegung des Betätigungselements 118 entlang der Achse bzw. Welle 160 (Figur 3) durchstößt der Sensor 122 mit seiner Spitze 150 das das Kompartiment 128 abdichtende Septum 134. Im Kompartiment 128 kann ein weiteres Kalibriermittel oder ein Reinigungsmittel aufgenommen sein, so dass der Sensor 122 bzw. dessen sensitives Element 146 in gewünschter Art kalibriert bzw. gereinigt werden kann.

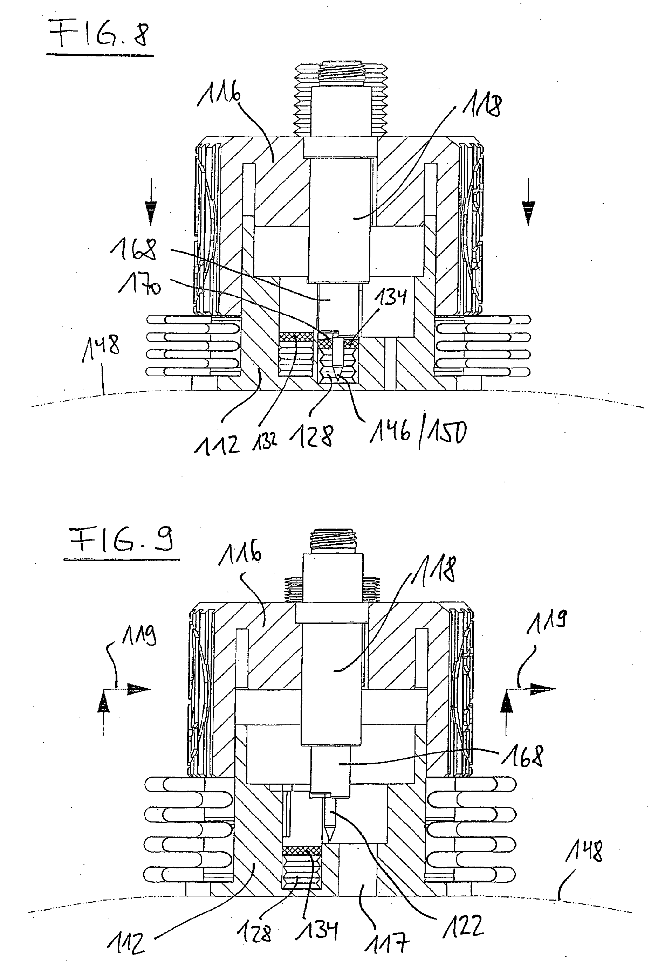

Nachdem der in Figur 8 dargestellte Kalibrierungs- bzw. Reinigungsschritt durchgeführt worden ist, kann das Betätigungselement 116 erneut in Richtung weg vom Fermenter 148 bewegt werden und anschließend im Gegenuhrzeigersinn weiter verdreht werden, bis der Sensor 122 im Wesentlichen mit der Durchgangsöffnung 117 fluchtet. Eine Zwischenposition während der Verdrehung ist der Figur 9 zu entnehmen, und in der Figur 10 ist die Messbereitschaftsposition dargestellt, in welcher das Betätigungselement vollständig zum Fermenter 148 hin nach unten durch die Öffnung 117 hindurch bewegt ist, so dass der Sensor 122 bzw. sein sensitives Element 146 im Innenraum (Reaktionsraum) des Fermenters 148 (Reaktionsbehälters) angeordnet ist.

Was hinsichtlich der Ausgestaltung der Kompartimente 126, 128 mit starren bzw. elastischen Wänden betrifft, wird auf die einleitenden Ausführungen hingewiesen.

Es wird ferner noch darauf hingewiesen, dass die Septen oder Abstreifelemente neben ihrer abdichtenden Funktion der Kompartimente bzw. der reinigenden Funktion für den Sensor auch eine den Sensor in seiner Position in einem jeweiligen Kompartiment stabilisierende Funktion aufweisen. Beispielhaft wird hier erneut auf Figur 1 und 2 verwiesen, denen ein oberhalb der Kompartimente 26, 28, 30 angeordneter Stützring 72 entnommen werden kann, welcher eine radiale Führung des relativ langen Sensors 122 insbesondere durch die ersten beiden Kompartimente 26 und 28 gewährleisten kann.

Mit einer Sensoreinheit 10 gemäß der ersten Ausführungsform (Figuren 1 und 2) wurden bereits erste Tests durchgeführt. Beim Test wurden zwei Kompartimente mit pH-Puffergelen pH4 bzw. pH7 gefüllt und eine pH-Einstab-Messkette mit kegelförmiger Glaskuppe verwendet. Die Viskositäten der beiden Kalibriergele wurden relativ hoch (> 5.000 mPa/s) gewählt. Das pH-Puffergel mit dem pH-Wert 4 diente als Aufbewahrungsgel. Die Septen der Kompartimente bestanden aus einer doppelschichtigen LDPE-Folie, die durch einen Schraubverschluss in ein Gewinde eingeschraubt wurde. Nach 30 Tagen der Lagerung wurde die pH-Einstab-Messkette gemäß dem unter Bezugnahme auf die Figuren 1 und 2 beschriebenen Vorgang in einer in den Figuren nicht dargestellten Überkopfposition in den beiden Kalibriergelen pH4-Puffergel und pH7-Puffergel kalibriert. Anschließend wurde eine pH-Messung zur Kontrolle in einem von den Kompartimenten getrennten außerhalb der Kalibriereinheit angeordneten pH4-Puffergel durchgeführt. Es konnte dabei eine sehr gute Übereinstimmung der Sollwerte mit den gemessenen pH-Werten festgestellt werden. Ferne erfolgte durch die Septen eine sehr gute Reinigung der pH-Elektroden (sensitives Element 46). Es konnten weder nennenswerte Gelrückstände am Sensor noch ein Austritt von Gel in den Reaktionsraum (Fermenter) festgestellt werden.

Die hier vorgestellten Sensoreinheiten 10, 110 können mit einem Einwegfermenter fest verbunden, insbesondere verschraubt oder verklebt oder verscheißt sein. Die Kalibriereinheiten und insbesondere deren Kalibriermittel bzw. Reinigungsmittel und Sensoren sind so ausgestaltet, dass der Fermenter zusammen mit daran angebrachten kalibrierbaren Sensoreinheiten zuverlässig sterilisiert werden kann. Somit können insbesondere herkömmliche und bewährte Sensoren bei Einwegfermentern zum Einsatz kommen, ohne dass der Fermenter beim Anbringen oder/und Kalibrieren der Sensoren kontaminiert wird.

## Patentansprüche

1. Kalibrierbare Sensoreinheit für einen Reaktionsbehälter, wie etwa einen Fermenter (48; 148), insbesondere Einwegfermenter, umfassend wenigstens eine zu kalibrierende Sensoreinrichtung (14; 114) und ein ein Kalibriermittel enthaltendes Kompartiment (26, 28, 30; 126, 128), wobei die wenigstens eine Sensoreinrichtung (14; 114) und das Kompartiment (26, 28, 30; 126, 128) relativ zueinander beweglich in einem Gehäuse (12; 112) aufgenommen sind, das mit dem Reaktionsbehälter (48; 148) verbindbar ist, wobei die kalibrierbare Sensoreinheit (10; 110) dazu eingerichtet ist, die wenigstens eine Sensoreinrichtung (14; 114) durch Kontakt mit dem Kalibriermittel zu kalibrieren bevor mit der wenigstens einen Sensoreinrichtung (14; 114) chemische oder/und physikalische Parameter einer Messsubstanz gemessen werden, wobei die wenigstens eine Sensoreinrichtung (14; 114) relativ zu dem Kompartiment (26, 28, 30; 126, 128) von einer Ausgangsposition, in welcher die Sensoreinrichtung (14; 114) bei bestimmungsgemäßem Gebrauch von der Messsubstanz nicht kontaktierbar ist, unter Änderung der Umgebung wenigstens eines Teils der Sensoreinrichtung (14; 114), insbesondere eines sensitiven Elements (46; 146), in eine von der Ausgangsposition verschiedene Messbereitschaftsposition beweglich ist, in welcher die Sensoreinrichtung (14; 114) wenigstens teilweise, insbesondere mit einem sensitiven Element (46; 146), von der Messsubstanz kontaktierbar ist,
**dadurch gekennzeichnet, dass** die kalibrierbare Sensoreinheit (10; 110) derart ausgebildet ist, dass die Relativbewegung zwischen der wenigstens einen Sensoreinrichtung (14; 114) und dem Kompartiment (26, 28, 30; 126, 128) irreversibel durchführbar ist.

2. Sensoreinheit nach Anspruch 1,
**dadurch gekennzeichnet, dass** die wenigstens eine Sensoreinrichtung (14; 114), insbesondere ihr sensitives Element (46; 146) in der Ausgangsposition in dem Kompartiment (26; 126) mit Kalibriermittel aufgenommen ist.

3. Sensoreinheit nach Anspruch 1 oder 2,
**dadurch gekennzeichnet, dass** das Gehäuse die wenigstens eine Sensoreinrichtung (14;114) in ihrer Ausgangsposition und das Kompartiment (26, 28, 30; 126, 128) umgibt.

4. Sensoreinheit nach einem der Ansprüche 1 bis 3,
**dadurch gekennzeichnet, dass** die wenigstens eine Sensoreinrichtung in der Messbereitschaftsposition selbsttätig gehalten, insbesondere verrastet ist.

5. Sensoreinheit nach einem der Ansprüche 1 bis 4,
**dadurch gekennzeichnet, dass** das Kompartiment (26, 28, 30; 126, 128) durch wenigstens ein Septum (32, 34, 36; 132, 134) verschlossen ist, insbesondere von dem durch das Gehäuse (12; 112) umschlossenen Innenraum der kalibrierbaren Sensoreinheit (10; 110) oder von einem benachbarten Kompartiment (28, 30) oder vom Fermenter (48; 148) getrennt ist.

6. Sensoreinheit nach Anspruch 5,
**dadurch gekennzeichnet, dass** das wenigstens eine Septum (32, 34, 36; 132, 134) für ein leichtes Durchdringen mittels der Sensoreinrichtung (14;114) vorbereitet ist, etwa zur Bildung einer Sollbruchstelle, insbesondere Bereiche unterschiedlicher Materialdicke aufweist oder/und wenigstens teilweise perforiert ist.

7. Sensoreinheit nach Anspruch 5 oder 6,
**dadurch gekennzeichnet, dass** die Sensoreinrichtung (14; 114) ein freies Ende (50; 150) aufweist, das derart ausgebildet ist, dass das wenigstens eine Septum (32, 34, 36; 132, 134) des wenigstens einen Kompartiments (26, 28, 30; 126, 128) durchstoßbar ist, vorzugsweise in einer zur Ebene des Septums (32, 34, 36; 132, 134) im Wesentlichen orthogonalen Richtung.

8. Sensoreinheit nach Anspruch 7,
**dadurch gekennzeichnet, dass** sie mehrere Kompartimente (26, 28, 30) umfasst, die bezogen auf die Durchstoßrichtung der Sensoreinrichtung (14) hintereinander angeordnet und durch jeweilige dazwischen angeordnete Septen (32, 34, 36) voneinander getrennt sind.

9. Sensoreinheit nach Anspruch 7 oder 8,
**dadurch gekennzeichnet, dass** sie mehrere Kompartimente (126, 128) umfasst, die im Wesentlichen orthogonal zur Durchstoßrichtung nebeneinander angeordnet sind.

10. Sensoreinheit nach Anspruch 8 oder 9,
**dadurch gekennzeichnet, dass** wenigstens eines der mehreren Kompartimente (28; 128) ein Reinigungsmittel enthält.

11. Sensoreinheit nach einem der Ansprüche 7 bis 10,
**dadurch gekennzeichnet, dass** dem Kompartiment (26, 28, 30; 126, 128) eine Abstreifeinrichtung (38, 40) zugeordnet ist, die von der wenigstens einen Sensoreinrichtung (14; 114) durchstoßbar ist und derart ausgebildet ist, dass an der wenigstens einen Sensoreinrichtung (14; 114) anfhaftende Verunreinigungen, insbesondere anhaftendes Kalibriermittel oder gegebenfalls Reinigungsmittel vorzugsweise im betreffenden Kompartiment (26, 28, 30; 126, 128) mit Kalibriermittel oder gegebenenfalls Reinigungsmittel zurückbehalten wird.

12. Sensoreinheit nach Anspruch 11,
**dadurch gekennzeichnet, dass** die Abstreifeinrichtung durch wenigstens ein Septum oder durch wenigstens ein zusätzliches in Durchstoßrichtung dem Septum benachbartes Abstreifmittel (38, 40), insbesondere Dichtring gebildet ist.

13. Sensoreinheit nach einem der Ansprüche 5 bis 12, **dadurch gekennzeichnet, dass** das Kompartiment (26, 28, 30; 126, 128) als rohrartige Hülse ausgebildet ist, die eine elastische oder steife Umfangswandung aufweist und in axialer Richtung wenigstens einseitig mit einem Septum (32, 34, 36; 132, 134) verschlossen ist.

14. Sensoreinheit nach einem der vorhergehenden Ansprüche,
**dadurch gekennzeichnet, dass** sie auf die wenigstens eine Sensoreinrichtung (14; 114) wirkende Betätigungsmittel (16; 116) umfasst, die derart ausgeführt sind, dass mittels der Betätigungsmittel (16; 116) die irreversible Relativbewegung zwischen der wenigstens einen Sensoreinrichtung (14; 114) und dem Kompartiment (26, 28, 30; 126, 128) durchführbar ist.

15. Sensoreinheit nach einem der vorhergehenden Ansprüche,
**dadurch gekennzeichnet, dass** das Kalibriermittel oder/und das Reinigungsmittel als sterilisierbares viskoses Medium ausgeführt ist, vorzugsweise als Gel oder Paste.

16. Sensoreinheit nach Anspruch 15,
**dadurch gekennzeichnet, dass** das wenigstens eine Kalibriermittel synthetische Polymere, insbesondere Polyvinylalkohol oder Hydroxyethylcellulose, oder natürliche Polymere, insbesondere Polysaccharide als Verdickungsmittel enthält.

17. Reaktionsbehälter, wie etwa Fermenter, insbesondere Einwegfermenter mit einem Reaktionsraum und einer damit fest verbundenen, insbesondere verschraubten oder verklebten oder verschweißten kalibrierbaren Sensoreinheit (10; 110) nach einem der vorhergehenden Ansprüche, wobei der Reaktionsbehälter (48; 148) und die kalibrierbare Sensoreinheit (10; 110) gemeinsam sterilisierbar oder sterilisert sind.

18. Reaktionsbehälter nach Anspruch 17,
**dadurch gekennzeichnet, dass** die Sensoreinrichtung (14; 114) in der Messbereitschaftsposition wenigstens teilweise, insbesondere mit ihrem sensitiven Element (46; 146), im Reaktionsraum aufgenommen ist.

19. Reaktionsbehälter nach Anspruch 17 oder 18,
**dadurch gekennzeichnet, dass** der Reaktionsraum durch wenigstens ein dem Reakionsbehälter, insbesondere dessen Hülle oder/und der kalibrierbaren Sensoreinheit (10; 110), zugeordnetes Septum von der kalibrierbaren Sensoreinheit (10; 110) getrennt ist.

20. Verfahren zum Kalibrieren wenigstens einer Sensoreinrichtung (14; 114) einer mit einem Reaktionsbehälter, wie etwa Fermenter (48; 148), insbesondere Einwegfermenter verbundenen kalibrierbaren Sensoreinheit (10; 110) nach einem der Ansprüche 1 bis 16, umfassend die Schritte:
a) Einführen eines sensitiven Elements (46; 146) der wenigstens einen Sensoreinrichtung (14; 114) in ein Kalibriermittel enthaltendes Kompartiment (26; 126) der kalibrierbaren Sensoreinheit (10; 110),
b) Herausführen des sensitiven Elements (46; 146) aus dem Kompartiment (26; 126),
c) Einführen des sensitiven Elements (46; 146) in den Reaktionsbehälter (48; 148) und Arretieren der Sensoreinrichtung (14; 114), wobei die Schritte a) bis c) irreversibel durchgeführt werden.

21. Verfahren nach Anspruch 20, wobei die Schritte a) und b) vor Ausführung des Schritts c) an wenigstens einem weiteren Kompartiment (28, 30; 128) durchgeführt werden, in dem ein weiteres Kalibriermittel oder ein Reinigungsmittel enthalten ist.

22. Verfahren nach Anspruch 21, wobei beim Wiederholen der Schritte a) und b) an jedem weiteren Kompartiment (28, 30; 128) die wenigstens eine Sensoreinrichtung (14; 114) so geführt, gegebenenfalls temporär arretiert wird, dass das sensitive Element (46; 146) nicht mehr in Kontakt mit einem vorhergehenden Kompartiment (26; 126) kommt.

## Claims

1. Calibratable sensor unit for a reaction vessel, such as a fermenter (48; 148), in particular a disposable fermenter, comprising at least one sensor device (14; 114) to be calibrated and a compartment (26, 28, 30; 126, 128) containing a calibrating agent, the at least one sensor device (14; 114) and the compartment (26, 28, 30; 126, 128) being received in a housing (12; 112) such that they can move relative to one another, it being possible to connect said housing to the reaction vessel (48; 148), the calibratable sensor unit (10; 110) being configured to calibrate the at least one sensor device (14; 114) by contact being made with the calibrating agent before chemical and/or physical parameters of a substance to be measured are measured by the at least one sensor device (14; 114), it being possible for the at least one sensor device (14; 114) to move, as a result of a change in the environment of at least a part of the sensor device (14; 114), in particular of a sensitive element (46; 146), relative to the compartment (26, 28, 30; 126, 128) from a starting position, in which, under normal conditions of use, the sensor device (14; 114) cannot come into contact with the substance to be measured, into a measurement-ready position which is different from the starting position and in which at least a part of the sensor device (14; 114), in particular a sensitive element (46; 146), can come into contact with the substance to be measured,
**characterised in that** the calibratable sensor unit (10; 110) is designed in such a way that the relative movement between the at least one sensor device (14; 114) and the compartment (26, 28, 30; 126, 128) can be carried out irreversibly.

2. Sensor unit according to claim 1, **characterised in that**, in the starting position, the at least one sensor device (14; 114), in particular the sensitive element (46; 146) thereof, is received in the compartment (26; 126) comprising calibrating agent.

3. Sensor unit according to either claim 1 or claim 2, **characterised in that** the housing surrounds the at least one sensor device (14; 114), when said sensor device is in its starting position, and the compartment (26, 28, 30; 126, 128).

4. Sensor unit according to any of claims 1 to 3, **characterised in that** the at least one sensor device is automatically held, in particular latched, in the measurement-ready position.

5. Sensor unit according to any of claims 1 to 4, **characterised in that** the compartment (26, 28, 30; 126, 128) is closed by means of at least one septum (32, 34, 36; 132, 134) and is in particular separated from the inner space, which is enclosed by the housing (12; 112), of the calibratable sensor unit (10; 110) or from an adjacent compartment (28, 30) or from the fermenter (48; 148).

6. Sensor unit according to claim 5, **characterised in that** the at least one septum (32, 34, 36; 132, 134) is prepared for simple penetration by means of the sensor device (14; 114), for example comprising in particular regions of varying material thickness and/or being perforated at least in part in order to form a predetermined breaking point.

7. Sensor unit according to either claim 5 or claim 6, **characterised in that** the sensor device (14; 114) has a free end (50; 150) which is designed in such a way that the at least one septum (32, 34, 36; 132, 134) of the at least one compartment (26, 28, 30; 126, 128) can be pierced, preferably in a direction which is substantially orthogonal to the plane of the septum (32, 34, 36; 132, 134).

8. Sensor unit according to claim 7, **characterised in that** it comprises a plurality of compartments (26, 28, 30) which are arranged one behind the other in relation to the piercing direction of the sensor device (14) and are separated from one another by septa (32, 34, 36) arranged between each compartment.

9. Sensor unit according to either claim 7 or claim 8, **characterised in that** it comprises a plurality of compartments (126, 128) arranged side by side in a direction substantially orthogonal to the piercing direction.

10. Sensor unit according to either claim 8 or claim 9, **characterised in that** at least one of the plurality of compartments (28; 128) contains a cleaning agent.

11. Sensor unit according to any of claims 7 to 10, **characterised in that** the compartment (26, 28, 30; 126, 128) is assigned a wiping device (38, 40) which can be pierced by the at least one sensor device (14; 114) and is designed such that impurities, in particular adherent calibrating agent or, if applicable, cleaning agent, adhering to the at least one sensor device (14; 114) are preferably retained in the respective compartment (26, 28, 30; 126, 128) comprising calibrating agent or, if applicable, cleaning agent.

12. Sensor unit according to claim 11, **characterised in that** the wiping device is formed by at least one septum or by at least one additional wiping means (38, 40), in particular a sealing ring, which is adjacent to the septum in the piercing direction.

13. Sensor unit according to any of claims 5 to 12, **characterised in that** the compartment (26, 28, 30; 126, 128) is formed as a tubular sleeve which has a resilient or rigid circumferential wall and is closed in the axial direction at least on one side by a septum (32, 34, 36; 132, 134).

14. Sensor unit according to any of the preceding claims, **characterised in that** it comprises actuating means (16; 116) which act on the at least one sensor device (14; 114) and are designed such that the irreversible relative movement between the at least one sensor device (14; 114) and the compartment (26, 28, 30; 126, 128) can be carried out by means of the actuating means (16; 116).

15. Sensor unit according to any of the preceding claims, **characterised in that** the calibrating agent and/or the cleaning agent is a sterilisable viscous medium, preferably a gel or paste.

16. Sensor unit according to claim 15, **characterised in that** the at least one calibrating agent contains either synthetic polymers, in particular polyvinyl alcohol or hydroxyethyl cellulose, or natural polymers, in particular polysaccharides, as thickening agents.

17. Reaction vessel, such as a fermenter, in particular a disposable fermenter, comprising a reaction chamber and a calibratable sensor unit (10; 110), according to any of the preceding claims, which is rigidly connected, in particular screwed or glued or welded, to said reaction chamber, wherein the reaction vessel (48; 148) and the calibratable sensor unit (10; 110) can be or are jointly sterilised.

18. Reaction vessel according to claim 17, **characterised in that**, when in the measurement-ready position, at least part of the sensor device (14; 114), in particular the sensitive element (46; 146) thereof, is received in the reaction chamber.

19. Reaction vessel according to either claim 17 or claim 18, **characterised in that** the reaction chamber is separated from the calibratable sensor unit (10; 110) by means of at least one septum assigned to the reaction vessel, in particular the casing thereof, and/or to the calibratable sensor unit (10; 110).

20. Method for calibrating at least one sensor device (14; 114) of a calibratable sensor unit (10; 110), according to any of claims 1 to 16, which is connected to a reaction vessel, such as a fermenter (48; 148), in particular a disposable fermenter, comprising the steps of:
a) inserting a sensitive element (46; 146) of the at least one sensor device (14; 114) into a compartment (26; 126) of the calibratable sensor unit (10; 110), which compartment contains calibrating agent,
b) guiding the sensitive element (46; 146) out of the compartment (26; 126),
c) inserting the sensitive element (46; 146) into the reaction vessel (48; 148) and locking the sensor device (14; 114), wherein steps a) to c) are carried out irreversibly.

21. Method according to claim 20, wherein steps a) and b) are carried out for at least one additional compartment (28, 30; 128) containing an additional calibrating agent or cleaning agent, before step c) is carried out.

22. Method according to claim 21, wherein, when steps a) and b) are repeated for each additional compartment (28, 30; 128), the at least one sensor device (14; 114) is guided, optionally temporarily locked, such that the sensitive element (46; 146) no longer comes into contact with a previous compartment (26; 126).

## Revendications

1. Unité de détection pouvant être calibrée pour un réservoir de réaction, tel qu'un fermenteur (48; 148), en particulier un fermenteur à usage unique, comprenant au moins un dispositif de détection à calibrer (14; 114) et un compartiment (26, 28, 30; 126, 128) contenant un moyen de calibrage, l'au moins un dispositif de détection (14; 114) et le compartiment (26, 28, 30; 126, 128) étant logés dans un boîtier (12; 112), qui peut être relié au réservoir de réaction (48; 148), en étant mobiles l'un par rapport à l'autre, l'unité de détection pouvant être calibrée (10; 110) étant conçue pour calibrer l'au moins un dispositif de détection (14; 114) par contact avec le moyen de calibrage avant que des paramètres chimiques et/ou physiques d'une substance à mesurer ne soient mesurés avec l'au moins un dispositif de détection (14; 114), l'au moins un dispositif de détection (14; 114) étant mobile relativement au compartiment (26, 28, 30; 126, 128) depuis une position de départ, dans laquelle le dispositif de détection (14; 114), moyennant une utilisation conforme, ne peut pas entrer en contact avec la substance à mesurer, vers une position de disponibilité pour la mesure différente de la position de départ, dans laquelle le dispositif de détection (14; 114) peut entrer en contact au moins partiellement, en particulier avec un élément sensitif (46; 146), avec la substance à mesurer lors d'une modification de l'environnement d'au moins une partie du dispositif de détection (14; 114), en particulier d'un élément sensitif (46; 146),
**caractérisée en ce que** l'unité de détection pouvant être calibrée (10; 110) est formée de telle manière que le mouvement relatif entre l'au moins un dispositif de détection (14; 114) et le compartiment (26, 28, 30; 126, 128) est réalisable de manière irréversible.

2. Unité de détection suivant la revendication 1,
**caractérisée en ce que** l'au moins un dispositif de détection (14; 114), en particulier son élément sensitif (46; 146) est dans la position de départ logé dans le compartiment (26; 126) avec le moyen de calibrage.

3. Unité de détection suivant la revendication 1 ou 2,
**caractérisée en ce que** le boîtier entoure l'au moins un dispositif de détection (14; 114) dans sa position de départ et le compartiment (26, 28, 30; 126, 128).

4. Unité de détection suivant l'une des revendications 1 à 3,
**caractérisée en ce que** l'au moins un dispositif de détection est maintenu, en particulier verrouillé, automatiquement dans la position de disponibilité pour la mesure.

5. Unité de détection suivant l'une des revendications 1 à 4,
**caractérisée en ce que** le compartiment (26, 28, 30; 126, 128) est fermé au moins par un septum (32, 34, 36; 132, 134), en particulier est isolé de l'espace intérieur entouré par le boîtier (12; 112) de l'unité de détection pouvant être calibrée (10; 110) ou d'un compartiment voisin (28, 30) ou du fermenteur (48; 148).

6. Unité de détection suivant la revendication 5,
**caractérisée en ce que** l'au moins un septum (32, 34, 36; 132, 134) est préparé pour une pénétration aisée à l'aide du dispositif de détection (14; 114), notamment par la formation d'un point de rupture, en particulier présente des zones d'épaisseurs de matériau différentes ou/et est au moins partiellement perforé.

7. Unité de détection suivant la revendication 5 ou 6,
**caractérisée en ce que** le dispositif de détection (14; 114) présente une extrémité libre (50; 150), qui est formée de telle façon que l'au moins un septum (32, 34, 36; 132, 134) de l'au moins un compartiment (26, 28, 30; 126, 128) peut être transpercé, de préférence dans une direction essentiellement orthogonale au plan du septum (32, 34, 36; 132, 134).

8. Unité de détection suivant la revendication 7,
**caractérisée en ce qu'**elle comprend plusieurs compartiments (26, 28, 30) qui sont disposés l'un derrière l'autre par rapport à la direction de transpercement du dispositif de détection (14) et sont séparés l'un de l'autre par des septums respectifs (32, 34, 36) disposés entre eux.

9. Unité de détection suivant la revendication 7 ou 8,
**caractérisée en ce qu'**elle comprend plusieurs compartiments (126, 128) qui sont disposés l'un à côté de l'autre essentiellement orthogonalement à la direction de transpercement.

10. Unité de détection suivant la revendication 8 ou 9,
**caractérisée en ce qu'**au moins un des plusieurs compartiments (28; 128) contient un produit de nettoyage.

11. Unité de détection suivant l'une des revendications 7 à 10,
**caractérisée en ce qu'**au compartiment (26, 28, 30; 126, 128) est affecté un dispositif de raclage (38, 40) qui peut être transpercé par l'au moins un dispositif de détection (14; 114) et est formé de telle façon que les impuretés adhérant à l'au moins un dispositif de détection (14; 114), en particulier du moyen de calibrage ou le cas échéant du produit de nettoyage qui adhèrent, sont de préférence retenues dans le compartiment correspondant (26, 28, 30; 126, 128) avec le moyen de calibrage ou le cas échéant le produit de nettoyage.

12. Unité de détection suivant la revendication 11,
**caractérisée en ce que** le dispositif de raclage est formé par au moins un septum ou par au moins un moyen de raclage (38, 40) supplémentaire, en particulier une bague d'étanchéité, voisin du septum dans la direction de transpercement.

13. Unité de détection suivant l'une des revendications 5 à 12,
**caractérisée en ce que** le compartiment (26, 28, 30; 126, 128) est formé comme une enveloppe tubulaire qui présente une paroi périphérique élastique ou rigide et est fermée en direction axiale au moins d'un côté par un septum (32, 34, 36; 132, 134).

14. Unité de détection suivant l'une des revendications précédentes, **caractérisée en ce qu'**elle comprend des moyens d'actionnement (16; 116) agissant sur l'au moins (14; 114) un dispositif de détection qui sont réalisés de telle manière que le mouvement irréversible entre l'au moins un dispositif de détection (14; 114) et le compartiment (26, 28, 30; 126, 128) peut être réalisé à l'aide des moyens d'actionnement (16; 116).

15. Unité de détection suivant l'une des revendications précédentes, **caractérisée en ce que** le moyen de calibrage ou/et le produit de nettoyage sont réalisés comme fluide visqueux stérilisable, de préférence un gel ou une pâte.

16. Unité de détection suivant la revendication 15,
**caractérisée en ce que** l'au moins un moyen de calibrage contient des polymères synthétiques, en particulier de l'alcool de polyvinyle ou de l'hydroxyéthylcellulose, ou des polymères naturels, en particulier des polysaccharides comme épaississant.

17. Réservoir de réaction, comme par exemple un fermenteur, en particulier un fermenteur à usage unique avec un espace de réaction et une unité de détection pouvant être calibrée (10; 110) reliée de manière fixe à celui-ci, en particulier vissée ou collée ou soudée suivant l'une des revendications précédentes, le réservoir de réaction (48; 148) et l'unité de détection pouvant être calibrée (10; 110) étant ou pouvant être stérilisés en commun.

18. Réservoir de réaction suivant la revendication 17,
**caractérisé en ce que** le dispositif de détection (14; 114) est dans la position de disponibilité pour la mesure au moins partiellement logé dans l'espace de réaction, en particulier avec son élément sensitif (46; 146).

19. Réservoir de réaction suivant la revendication 17 ou 18,
**caractérisé en ce que** l'espace de réaction est séparé de l'unité de détection pouvant être calibrée (10; 110) par au moins un septum affecté au réservoir de réaction, en particulier à son enveloppe ou/et à l'unité de détection pouvant être calibrée (10; 110).

20. Procédé de calibrage d'au moins un dispositif de détection (14; 114) d'une unité de détection pouvant être calibrée (10; 110) suivant l'une des revendications 1 à 16 reliée à un réservoir de réaction, par exemple un fermenteur (48; 148), en particulier un fermenteur à usage unique, comprenant les étapes:
a) l'introduction d'un élément sensitif (46; 146) de l'au moins un dispositif de détection (14; 114) dans un compartiment (26; 126) contenant un moyen de calibrage de l'unité de détection pouvant être calibrée (10; 110),
b) l'amenée de l'élément sensitif (46; 146) hors du compartiment (26; 126),
c) l'introduction de l'élément sensitif (46; 146) dans le réservoir de réaction (48; 148) et le blocage du dispositif de détection (14; 114), les étapes a) à c) étant exécutées de manière irréversible.

21. Procédé suivant la revendication 20, les étapes a) et b) étant exécutées avant l'exécution de l'étape c) sur au moins un autre compartiment (28, 30; 128), dans lequel un autre moyen de calibrage ou un produit de nettoyage est contenu.

22. Procédé suivant la revendication 21, l'au moins un dispositif de détection (14; 114) étant guidé, le cas échéant temporairement bloqué, lors de la répétition des étapes a) et b) sur chaque compartiment supplémentaire (28, 30; 128) de telle façon que l'élément sensitif (46; 146) ne vient plus en contact avec un compartiment précédent (26; 126).
